# EUROPÄISCHE PATENTSCHRIFT

(11) **EP 1 677 766 B1**
(45) Veröffentlichungstag und Bekanntmachung des Hinweises auf die Patenterteilung: **14.03.2012**
(21) Anmeldenummer: 04790997.3
(22) Anmeldetag: 28.10.2004
(51) Int. Cl.: A61K 9/16, A61K 9/36, A61K 9/26, A61K 9/62

(54) **PHARMAZEUTISCHE WIRKSTOFFHALTIGE FORMULIERUNG MIT ÜBERZUG**
PHARMACEUTICAL AGENT-CONTAINING FORMULATION COMPRISING A COATING
PREPARATION PHARMACEUTIQUE CONTENANT DES SUBSTANCES ACTIVES ET PRESENTANT UN ENROBAGE

(30) Priorität: 31.10.2003 DE 10351301; 24.03.2004 DE 102004014828
(43) Veröffentlichungstag der Anmeldung: 12.07.2006
(73) Patentinhaber: Hexal AG, 83607 Holzkirchen (DE)
(72) Erfinder: KLOKKERS, Karin, 83620 Feldkirchen-Westerham (DE); ZELLNER, Marion, 85658 Egmating (DE); RILLMANN, Thomas, 76753 Bellheim (DE); DAUER, Andreas, 83052 Heufeld (DE)
(74) Vertreter: Forstmeyer, Dietmar
(86) Internationale Anmeldenummer: PCT/EP2004/012230
(87) Internationale Veröffentlichungsnummer: WO 2005/041934

(56) Entgegenhaltungen:
- WO-A-00/25856
- WO-A-96/37195
- WO-A-02/058677
- WO-A-03/051340
- DE-A1- 3 900 811
- DE-A1- 19 905 906
- US-A1- 2003 133 983

## Beschreibung

Die Erfindung betrifft ein Verfahren zur Herstellung einer wässerrigen Dispersion zur Herstellung eines Überzuges und ein Verfahren zur Herstellung einer wirkstoffhaltigen Formulierug zu oraler Verabreichung, die mit einem filmbildendem Polymeren überzogen ist.

Eine orale Verabreichung von Wirkstoffen zeigt eine gute Patienten-Compliance. Je nach Eigenschaften des Wirkstoffs und gewünschtem Freisetzungsprofil wird eine entsprechende Galenik, beipielsweise eine modifiziert freisetzende Arzneiform entwickelt. Hierzu zählen die verzögert freisetzenden oder retardiert freisetzenden Formulierungen.

Bei der Herstellung von verzögert freisetzenden Arzneiformen können Tabletten oder Pellets mit magensaftresistenten, dünndarmlöslichen Filmen überzogen werden.

Bei den Retard-Formulierungen unterscheidet man zwischen Matrix-Systemen, wobei der Wirkstoffe mit einer retardierenden Matrix (Polymer, Wachs) vermischt wird, und Reservoir-Systemen, wobei ein wirkstoffhaltiger Kern (z.B. Tablette oder Pellet) mit einem Polymerfilm überzogen wird. Der retardierende Überzug umhüllt den Wirkstoff und erlaubt so eine graduelle Freisetzung.

Als Retard-Formulierung werden bevorzugt multiple-unit-dosage-forms eingesetzt. Eine multiple-unit-dosage-form kann eine Tablette sein, die rasch im Magen zerfällt und eine Vielzahl überzogener Einheiten (Pellets) freisetzt. Sie kann auch als eine mit Pellets gefüllte Kapsel vorliegen.

Ein Vorteil einer Retard-Formulierung ist das gleichmäßige und langanhaltende effektive Wirkstoffniveau. Der zeitliche Abstand zwischen den einzelnen Tabletteneinnahmen ist für retardierte Arzneiformen größer als bei schnellfreisetzenden Formulierungen. So kann eine bessere Patienten-Compliance erreicht werden.

Vorteile einer multiple-unit-dosage-form sind:
- geringes Risiko von "dose dumping"
- dispergierte Wirkstoffdosis, d.h. das Risiko lokaler Irritationen ist gering
- in vivo Verhalten: geringe Schwankungen bei der Freisetzung im Magen, wodurch eine reproduzierbare Wirkstoff-Absorption gewährleistet ist
- die kleinen Pellets können mit Nahrungsmitteln gemischt werden und so die Einnahme erleichtern, insbesondere für ältere Patienten

Bisher werden die Polymerüberzüge überwiegend aus organischer Lösung auf die Pellets oder Tabletten aufgesprüht. Aus Umweltschutzgründen ist es jedoch notwendig, zu filmbildenden Polymeren auf Wasserbasis überzugehen.

Als filmbildende Polymere auf Wasserbasis eignen sich Polyacrylate.

Mit dem Begriff "Polyacrylat" werden Polymerisate auf Basis von Acrylsäure, Methacrylsäure, Acrylatester und/oder Methacrylatester bezeichnet. Polyacrylate sind unter den Handelsnamen "Eudragit" der Fa. Röhm und "Kollicoat" der Fa. BASF erhältlich.

In der Literatur sind folgende Methoden für das Überziehen von Pellets oder Tabletten mit wässrigen Polymerdispersionen beschrieben.

Gemäß EP 0 403 959 A1 sowie "Glyceryl monostearate as a glidant in aqueous film-coating formulations", H.-U. Petereit et. al., Eur. J. Pharm. 41 (4) 219-228, (1995) erfolgt das Überziehen von Kernen (Pellets, Tabletten) mit wässrigen Polymer-Dispersionen auf Acrylatbasis mit Hilfe lipophiler Emulgatoren wie Glycerinmonostearat, welche ein Verkleben der Pellets verhindern und stabile wässrige Dispersionen mit den Polymeren bilden.

In "Coating of Ibuprofen crystals with Eudragit FS30D", S. Schmid, Arch. Pharm. Med. Chem. 333, Suppl. 1, 2000, 1-40, Nr. 78 wird ein Verkleben der Partikel dadurch vermieden, daß Glycerylmonostearat als Trennmittel und Polysorbat 80 als Netzmittel zu der Eudragit FS30D Polymerdispersion zugesetzt werden. Die Filmqualität ist vom Gewichtsanteil Glyerylmonostearat und Polysorbat abhängig.

"Microencapsulated Eudragit RS30D coated controlled-release pellets: the influence of dissolution variables and topographical evaluation", T. Govender, J. Microencapsulation, Vol. 14, No. 1, 1997, S. 1-13 beschreibt die Verwendung von Magnesiumstearat als "Antiklebemittel" für das Überziehen von Kernen (Durchmesser ca. 1.9 mm) mit Eudragit RS30D. Die Wirksoff-Freisetzung basiert auf einer Kinetik erster Ordnung.

In US 5,529,790 wird das Erzielen einer bestimmten Freisetzungsrate der Wirkstoff-Pellets durch Verwendung wässriger Polymerdispersionen wie Eudragit NE oder Eudragit RS mit Additiven offenbart. Als Additive zur Kontrolle der Permeabilität dient insbesondere Magnesiumstearat in Kombination mit Citronensäure oder Simethicon. Magnesiumstearat verhindert auch ein Agglomerieren der Kerne (500 bis 1500 microns) während des Überziehens.

In "Eudragit RL and RS Pseudolatices: properties and performance in pharmaceutical coating as a controlled release membrane for theophyline pellets", R.-K. Chang, Drug Development and Industrial Pharmacy, 15 (2), 1989, S. 187-196 werden für das Überziehen von Theophyllin-Pellets mit Eudragit RL- und/oder RS-Pseudolatices als Trennmittel Talk und Siliciumdioxid verwendet. Für die Herstellung der Eudragit-Dispersion werden auch organische Lösungsmittel eingesetzt.

"Effect of application temperature on the dissolution profile of sustained-release theophylline pellets coated with Eudragit RS30D, P. Schmidt, F. Niemann, Drug Development and Industrial Pharmacy, 19 (13), 1603-1612 (1993) beschreibt das Überziehen von Theophyllin Pellets (100 bis 1400 µm) in einem "miniature fluid-bed pan coater" mit Eudragit RS30D, verschiedenen Weichmachern (Triethylcitrat, Dibutylphthalat, PEG) und Talk.

"Drug release from compressed Eudragit RS30D coated beads", G.F. Palmieri, S.T.P. Pharma Sciences 6 (2), 1996, S. 118-121, betrifft das Überziehen von Theophyllin-haltigen Kernen (Durchmesser 200 bis 630 µm) mit Eudragit RS30D, 20 % Triethylcitrat (Weichmacher) sowie 50 % Talk (bezogen auf das Tockengewicht des Polymeren), welcher zur Verringerung der Klebeneigung des Eudragit RS30D eingesetzt wird. Die mit dem Wirkstoffgranulat gefüllten Kapseln zeigen eine Freisetzung nullter Ordnung, die zu Tabletten verpressten Granulate zeigen je nach Granulatgehalt eine Freisetzung nullter bis erster Ordnung.

In "Influence of platicizer concentration and storage conditions on the drug release rate from Eudragit RS30D filmcoated sustained release theophylline pellets", K. Amighi, A. Moes, Eur. J. Pharm. Biopharm. 42 (1) 29-35 (1996) wird das Überziehen von Theophyllin Pellets (Durchmesser 1100 µm) mit Eudragit RS30D mit Hilfe von HPMC, Talk, Triethylcitrat (Weichmacher) sowie Silicon-Emulsion (Antischaummittel) beschrieben.

In "Effect of pectinolytic enzymes on the theophylline release from pellets coated with water insoluble polymers containing pectin HM or calcium pectinate", R. Semdé, Int. J. Pharm., 197, 2.000, S. 169-179, ebenso in "Influence of curing conditions on the drug release rate from Eudragit NE30D film coated sustained-release theophylline pellets", K. Amighi, S.T.P. Pharma Sciences 7 (2), 1997, S. 141-147 werden Talk und eine Silicon-Emulsion (Antischaummittel) als Hilfsstoffe beim Überziehen von Pellets (Durchmesser 1 mm) mit Eudragit NE30D eingesetzt, wobei der Film auch Pektin enthält. Eudragit RS30D wird unter Verwendung einer Silicon-Emulsion verarbeitet.

In "A pH-dependent colon targeted oral drug delivery system using methacrylic acid copolymers", M. Z. I. Khan, Journal of Controlled Release 58 (1999), 215-222, wird das Überziehen von Tabletten mit Eudragit S100 und Eudragit L 100-55 oder deren Mischungen aus wässriger Dispersion mit Triethylcitrat als Weichmacher und Talk als Gleitmittel beschrieben.

In "Modifying the release properties of Eudragit L30D", N. A. Muhammad, et. al., Drug development and industrial pharmacy, 17(18), 2497-2509 (1991), wird das Überziehen von wirkstoffhaltigen Kernen mit einer wässrigen Dispersion aus Eudragit L30D, Triethylcitrat und Kaolin in einem Glatt GPCG3 mit anschließendem "curing" der Pellets bei 45 °C beschrieben.

US 20020160046 beschreibt eine Retard-Formulierung für Omeprazol, wobei Omeprazol-haltige Kerne mit einem "dicken" retardierenden Überzug (100 bis 5000 microns) beispielsweise mit nicht-enterischen Formen von Eudragit versehen sind. Omeprazol-Kerne werden mit einer wässrigen Dispersion aus Eudragit NE30D, Talk, Magnesiumstearat, Glycerinmonostearat und Triethylcitrat besprüht. Der Überzug enthält hier das Tensid Glycerinmonostearat.

WO 99/12524 beschreibt die Herstellung von multiple-unit-dosage forms für NSAIDs. Wirkstoffhaltige Kerne werden beispielsweise mit einer Mischung aus Eudragit NE, Hypromellose, Talk und Magnesiumstearat überzogen. Um ein Verkleben der Pellets bei erhöhter Temperatur zu verhindern, wird ein zweiter Überzug mit einem filmbildenden Polymer aufgebracht.

EP 0 520 119 A1 offenbart Diclofenac-Pellets, die mit einer Membranschicht, welche Eudragit NE, Talk, Magnesiumstearat, Polysorbat und Silicon-Antischaumemulsion enthält, überzogen sind.

WO 03/051340 beschreibt eine Zubereitung von Metoprololsuccinat-Pellets, die mit einer Filmdispersion überzogen sind, die Eudragit NE30D, Kollicoat SR30D, Wasser und einen Stabilisator enthält.

In "Aqueous polyacrylate dispersions as coating materials for sustained and enteric release systems", D. Wouessidjewe, S.T.P. Pharma Sciences 7 (6), 1997, S. 469-475 wird das Überziehen mit Eudragit NE30D beschrieben. Eburnamonin-haltige Kerne (Durchmesser ca 1000 µm) werden mit einer wässrigen Dispersion von Eudragit NE30D und 10 % Talk als Trennmittel besprüht. Eine hohe Klebeneigung von Eudragit NE30D wird trotz der Verwendung von Talk beobachtet. Um ein Verkleben der Pellets beim Besprühen mit der Eudragit-Dispersion zu verhindern, wird ein diskontinuierliches Sprühverfahren angewendet, bei dem die Pellets abwechselnd besprüht und getrocknet werden. Die Prozesszeit beträgt im Labormaßstab mehr als 9 Stunden, d.h. dieses Verfahren ist sehr zeitintensiv.

In der Literatur wird Talk als bekanntes Antiklebemittel für wässrige Poly(meth)acrylat-Dispersionen beschrieben. Jedoch sedimentiert Talk in einer wässrigen Suspension sehr rasch, d.h. die Überzugsmitteldispersionen müssen kurz vor der Anwendung frisch zubereitet und während des Sprühens ständig gerührt werden. Magnesiumstearat wird seltener als Antiklebemittel verwendet. Nachteilig ist das Aufschwimmen von Magnesiumstearat in wässrigen Dispersionen, wodurch die Ausführung eines Sprühprozesses kompliziert wird.

Für die Verarbeitung von Eudragit NE30D wird vom Hersteller (Fa. Röhm) die Verwendung von Glycerolmonostearat oder Talkum empfohlen. Mikronisiertes Talkum wird dabei in einer Menge von bis 100 % bezogen auf die Polymermasse eingesetzt. Versuche zum Überziehen von wirkstoffhaltigen Kernen mit Eudragit NE30D - Talkum-Suspensionen in Wirbelschichtgeräten unterschiedlicher Maschinenkonfigurationen waren erfolglos. Nach Auftrag von nur 20 % des Polymeren kam es zu einem Verkleben der Mikropellets und damit zu einem Prozesszusammenbruch.

Aufgabe der Erfindung ist die Bereitstellung eines Verfahrens zur Herstellung einer pharmazeutischen wirkstoffhaltigen Formulierung zu oraler Verabreichung, vorzugsweise auf Wasserbasis, mit verbesserter Verarbeitbarkeit, welche eine geringe Klebrigkeit, hohe mechanische Stärke und Reproduzierbarkeit während des Verarbeitungsprozesses aufweist und zudem keine weiteren Überzüge oder Stabilisatoren wie Tenside oder Antischaummittel benötigt. Auch ein Verfahren zur Herstellung von überzogenen Formulierungen, wie Pellets oder Tabletten, soll vorgesehen werden, wobei die Herstellung kostengünstig und zeitsparend sein soll. Die überzogenen Formulierungen können ein modifiziertes Freisetzungsprofil für den (die) Wirkstoff(e) zeigen, insbesondere eine konstante Freisetzung (Nullte Ordnung).

Die der Erfindung zugrundeliegende Aufgabe wird gelöst durch die Bereitstellung des Verfahrens zur Herstellung einer wässerigen Dispersion zur Herstellung eines Überzuges gemäß Anspruch 1 und durch die Bereitstellung des Verfahrens zur Herstellung einer pharmazeutischen wirkstoffhaltigen Formulierung gemäß Anspruch 12. Bevorzugte Ausführungsformen der Erfindung werden durch die abhängigen Ansprüche bestimmt.

Bei der Formulierung kann der Überzug kein Tensid oder Antischaummittel als Stabilisator umfassen.

Ferner kann im erfindungsgemäßen Verfahren das filmbildende Polymere dadurch gekennzeichnet sein, dass es als Dispersion auf Wasserbasis vorgesehen werden kann.

Ferner kann es sich im erfindungsgemäßen Verfahren bei dem filmbildenden Polymeren um ein Gemisch von filmbildenden Polymeren handeln.

Im erfindungsgemäßen Verfahren kann ferner ein Polyacrylat als filmbildendem Polymeren vorgesehen sein.

Ferner kann es sich im erfundungsgemäßen Verfahren bei dem Polyacrylat um ein Polymerisat auf Basis von Acrylsäure, Methacrylsäure, Acrylsäureester und/oder Methacrylsäureester handeln, insbesondere Eudragit und/oder Kollicoat.

Ferner kann die Mischung mit den mindestens zwei Trennmitteln
- mindestens ein Trennmittel, das in reinem Wasser aufschwimmt, und
- mindestens ein Trennmittel umfassen, das in reinem Wasser absinkt oder sich darin löst.

Die Tatsache, ob ein Trennmittel in reinem Wasser aufschwimmt oder nicht aufschwimmt, muß nicht allein davon abhängen, ob die Dichte des Trennmittels größer als die des Wassers ist oder nicht. So hat beispielsweise Magnesiumstearat eine wahre Dichte von 1,09 g/cm³, schwimmt jedoch in Wasser auf.

Erfindungsgemäß umfasst die Mischung mit den mindestens zwei Trennmitteln
- mindestens ein Fettsäuresalz als Trennmittel und
- mindestens ein Silikat aus der durch Schichtsilikate gebildeten Gruppe als Trennmittel umfassen.

Ferner kann im erfindungsgemäßen Verfahren die Mischung als Fettsäuresalz ein Alkalisalz und/oder ein Erdalkalisalz und/oder ein Aluminiumsalz einer Fettsäure umfassen.

Ferner kann im erfindungsgemäßen Verfahren die Mischung Natrium-, Kalium-, Magnesium-, und/oder Calciumbehenat als Alkali- bzw. Erdalkalisalz einer Fettsäure umfassen.

Ferner kann im erfindungsgemäßen Verfahren die Mischung Natrium-, Kalium-, Magnesium-, Calcium- und/oder Aluminiumstearat als Alkali-, Erdalkali- bzw. Aluminiumsalz einer Fettsäure umfassen.

Ferner kann im erfindungsgemäßen Verfahren die Mischung ein Magnesiumsalz der Caprylsäure, Caprinsäure, Laurinsäure und/oder Palmitinsäure als Erdalkalisalz einer Fettsäure umfassen.

Ferner kann im erfindungsgemäßen Verfahren der Gehalt an Fettsäuresalz 5 bis 40 Gew.-% betragen, vorzugsweise 10 bis 30 Gew.-%, jeweils bezogen auf das Trockengewicht des filmbildenden Polymeren.

Ferner kann im erfindungsgemäßen Verfahren die Mischung als Schichtsilikat Talk, Kaolinit, Pyrophyllit, Attapulgit, Sepolit, Muskovit, Montmorillonit, Bentonit und/oder Vermiculit umfassen.

Ferner kann im erfindungsgemäßen Verfahren der Gehalt an Silikat 20 bis 60 Gew.-% betragen, vorzugsweise 30 bis 50 Gew.-%, jeweils bezogen auf das Trockengewicht des filmbildenden Polymeren.

Ferner können im erfindungsgemäßen Verfahren wirkstoffhaltige Kerne mit einem Überzug verschen werden, bei denen es sich um Kapseln, Tabletten, Pellets, Granulate, Minitabletten oder Mikropellets handelt.

Ferner können wirkstoffhaltige kerne verwendet werden, bei denen es sich um Wirkstoff-Kristalle handelt.

Ferner kann im erfindungsgemäßen Verfahren ein Pellet oder Mikropellet als wirkstoffhaltiger Kern einen inerten Kern (Inert-Kern) umfassen, wobei insbesondere ein inerter Kern mit einem wirkstoffhaltigen Überzug zu einem wirkstoffhaltigen Kern ausgebildet ist.

Ferner können im erfindungsgemäßen Verfahren die Mikropellets als Multiple-Unit-Dosage-Form vorgesehen sein, insbesondere in Form von Tabletten oder in Kapseln.

Ferner können im erfindungsgemäßen Verfahren die Pellets, Granulate oder Minitabletten als Multiple-Unit-Dosage-Form vorgesehen sein, insbesondere in Kapseln.

Ferner kann im erfindungsgemäßen Verfahren die Multiple-Unit-Dosage-Form ihrerseits mit einem erfindungsgemäßen Überzug versehen sein.

Ferner kann es sich im erfindungsgemäßen Verfahren bei der Multiple-Dosage-Form um eine Kapsel handeln, insbesondere eine Weichgelatine-Kapsel.

Ferner kann im erfindungsgemäßen Verfahren der Wirkstoff im Gemisch mit pharmazeutisch akzeptablen Hilfsstoffen vorgesehen sein, insbesondere mit üblichen Hilfsstoffen.

Ferner kann im erfindungsgemäßen Verfahren der Wirkstoff im Gemisch mit Tensiden, insbesondere nicht-ionischen oder ionischen oberflächenaktiven Substanzen, vorgesehen oder frei von Tensiden sein.

Ferner kann eine Formulierung mit einem gut wasserlöslichen Wirkstoff vorgesehen sein, vorzugsweise einer Löslichkeit von mehr als 300 g/l wässrige Lösung.

So kann die Formulierung mit Metoprolol oder einem seiner Salze als Wirkstoff vorgesehen sein, insbesondere Metoprolol-succinat.

Gemäß einer weiteren Ausführungsform wird die der Erfindung zugrundeliegende Aufgabe durch ein Verfahren zur Herstellung einer wässerigen Dispersion zur Herstellung eines Überzuges für eine pharmazeutische wirkstoffhaltige Formulierung zu oraler Verabreichung gemäß der Erfindung gelöst, wobei die Dispersion einen Gehalt an einem filmbildenden Polymeren und an mindestens zwei Trennmitteln aufweist und frei von Stabilisatoren ist, wobei
- mindestens ein Trennmittel, das in reinem Wasser aufschwimmt, in einer Menge von 5 bis 40 Gew.-% enthalten ist und
- mindestens ein Trennmittel, das in reinem Wasser absinkt, in einer Menge von 20 bis 60 Gew.-% enthalten ist, jeweils bezogen auf das Polymer-Trockengewicht.

Ferner wird die der Erfindung zugrundeliegende Aufgabe gemäß einer weiteren Ausführungsform durch ein Verfahren zur Herstellung einer wässrigen Dispersion zu Herstellung eines Überzuges für eine pharmazeutische wirkstoffhaltige Formulierung zu oraler Verabreichung gemäß der Erfindung gelöst, wobei die Dispersion einen Gehalt an einem filmbildenen Polymeren und an mindestens zwei Trennmitteln aufweist und frei von Stabilisatoren ist, wobei
- mindestens ein Fettsäuresalz als Trennmittel in einer Menge von 5 bis 40 Gew.-% enthalten ist und
- mindestens ein Silikat aus oder durch Schichtsilikat gebildeten Gruppe in einer Menge von 20 bis 60 Gew.-% enthalten ist, jeweils bezogen auf das Polymer-Trockengewicht.

Gemäß der Erfindung kann die Dispersion kein Tensid oder Antischaummittel als Stabilisator umfassen,
- insbesondere kein nicht-ionisches Tensid, besonders kein Polysorbat, Sorbitan-monoisostearat, Sorbitan-monolaurat, Sorbitan-monopalmitat, Sorbitan-monostearat, Sorbitanmonooleat, Sorbitan-sesquioleat, Sorbitan-trioleat, Glyceryl-monoetearat, Glyceryl-monooleat und/oder Polyvinylalkohol,
- insbesondere kein anionisches Tensid, besonders kein Natriumdocusat und/oder Natriumlaurylsulfat,
- insbesondere kein kationisches Tensid, besonders kein Benzalkoniumchlorid, Benzethoniumchlorid und/oder Cetrimid,
- insbesondere kein Antischaummittel auf Siliconbasis und/oder
- insbesondere kein Glycerol, Sorbitol und/oder PEG-Derivat als Antischaummittel.

Schließlich wird die der Erfindung zugrundeliegende Aufgabe gemäß einer weiteren Ausführungsform durch ein Verfahren zur Herstellung einer pharmazeutischen wirkstoffhaltigen Formulierung gemäß der Erfindung gelöst, bei dem man auf einer noch überzugsfreien Formulierung mit Hilfe einer erfindungsgemäßen Dispersion einen Überzug vorsieht.

Überraschenderweise wurde also unter anderem gefunden, dass eine gute Verarbeitbarkeit von wässrigen Polymer-Dispersionen für das Überziehen von Kernen (z.B. Pellets oder Tabletten) erreicht werden kann, wenn man den wässrigen Dispersionen des/der Polymeren eine Mischung aus mindestens zwei Trennmitteln zusetzt. Hierbei kann mindestens ein Trennmittel ein Alkali-, Erdalkali- oder Aluminiumsalz einer Fettsäure sein und mindestens ein weiteres Trennmittel ein Schichtsilikat. Die Klebeneigung des/der Polymeren wird durch die Verwendung einer Mischung aus mindestens zwei Trennmitteln herabgesetzt. Durch die Mischung mindestens zweier Trennmittel sehr unterschiedlicher Dichte wird offensichtlich eine Annäherung an die Dichte von Wasser erreicht. In Wasser tritt weder eine Sedimentation des Schichtsilikats noch eine Schaumbildung des Fettsäuresalzes auf. Eine Verwendung von Tensiden oder Antischaummitteln ist nicht notwendig.

Durch die Verwendung zweier Trennmittel werden die wirkstoffhaltigen Kerne lipophilisiert, wodurch eine Verlangsamung der Freisetzung und damit eine Kinetik nahezu nullter Ordnung erzielt werden kann.

Sofern das erfindungsgemäße Überziehen von wirkstoffhaltigen Kernen mit Hilfe von wässrigen Polymer-Dispersionen erfolgt, ist dieses Verfahren kostengünstiger als Prozesse, bei denen organische Lösungsmittel zur Verarbeitung des/der Polymer(en) eingesetzt werden. Teure, explosionsgeschütze Anlagen zum Überziehen der wirkstoffhaltigen Kerne wie bei Verfahren, die mit Hilfe organischer Lösungsmittel erfolgen, sowie die teure Entsorgung dieser Lösungsmittel sind entbehrlich. Zudem ist das erfindungsgemäße Herstellungsverfahren zeitsparend, da das Überziehen der wirkstoffhaltigen Kerne mit Hilfe einer Mischung aus mindestens zwei Trennmitteln mit einer hohen Sprührate und ohne Zwischentrocknungsschritte erfolgen kann.

Die Erfindung bezieht sich also unter anderem auf ein Verfahren zur Herstellung von Formulierungen wie Tabletten oder Pellets mit einem einzigen Filmüberzug, wobei der Filmüberzug aus einer wässrigen Dispersion des filmbildenden Polymeren aufgebracht wird. Das Überziehen mit der wässrigen Polymerdispersion erfolgt erfindungsgemäß mit Hilfe einer Mischung aus mindestens 2 Trennmitteln, insbesondere einem Alkali- oder Erdalkalisalz einer Fettsäure und einem Schichtsilikat. Der Filmüberzug ist frei von Stabilisatoren wie Tensiden oder Antischaummitteln.

Die Erfindung beschreibt also unter anderem ein filmbildendes System zur Herstellung modifiziert freisetzende Formulierungen mit einem einzigen Überzug umfassend:
- eine Polymerdispersion auf Wasserbasis,
- mindestens ein Fettsäuresalz als Trennmittel, wie beispielsweise Alkali- oder Erdalkalisalze von Fettsäuren,
- und mindestens ein Schichtsilikat als weiteres Trennmitteln, wobei die mindestens zwei Trennmittel gemischt werden und diese Mischung der Polymerdispersion zugesetzt wird.

Mit dem erfindungsgemäßen filmbildenden System können wirkstoffhaltige Kerne mit einer einzigen polymerhaltigen Schicht überzogen werden. Die Kerne können eine pharmakologisch wirksame Substanz und ggf. ein oder mehrere pharmazeutisch verträgliche Hilfsstoffe enthalten.

Wirkstoffe mit einer guten Wasserlöslichkeit werden besonders bevorzugt, da mit anderen Methoden nur schwer eine langanhaltende Freisetzung erreicht werden kann. Die Wasserlöslichkeit des Wirkstoffs beträgt bevorzugt mehr als 300 g/l.

Als Beispiele für wasserlösliche Wirkstoffe seien genannt: beta-Blocker, wie Metoprolol, Bisoprolol; Opioide, wie Tramadol, Morphin, Oxycodon oder Hydrocodon. Die Wirkstoffe können als Stereoisomere oder pharmazeutisch unbedenkliche Salze, Hydrate, Solvate sowie in Form von Derivaten eingesetzt werden. Es können auch Kombinationen aus zwei oder mehreren Wirkstoffen eingesetzt werden. Bevorzugt wird Metoprolol oder dessen Salze wie Tartrat, Succinat, Fumarat, Benzoat oder Sorbat verwendet. Das S-Enantiomer von Metoprolol oder dessen Benzoat- oder Sorbat-Salz kann ebenfalls eingesetzt werden. Besonders bevorzugt wird Metoprololsuccinat verwendet.

Die wirkstoffhaltigen Kerne können in Form von Tabletten, Pellets, Minitabletten, Granulaten oder Mikropellets vorliegen. Die überzogenen Pellets oder Minitabletten können in Kapseln gefüllt werden. Die überzogenen Mikropellets können zu Tabletten oder Kapseln, d.h. zu multiple-unit-dosage forms, weiterverarbeitet werden. Auch Wirkstoff-Kristalle und Kapseln, beispielsweise Weichgelatine-Kapseln, lassen sich mit dem erfindungsgemäßen filmbildenden System überziehen.

Die überzogenen wirkstoffhaltigen Kerne zeigen eine modifizierte Freisetzung. Bevorzugt wird der Wirkstoff über einen längeren Zeitraum, beispielsweise über 10 bis 24 Stunden freigesetzt.

### Wirkstoffhaltige Kerne:

Träger bzw. Kerne für die Überzüge können Kapseln, Tabletten, Granulate, Pellets oder Kristalle sein. Die Größe von Granulaten, Pellets oder Kristallen kann zwischen liegt 0,01 und 2,5 mm liegen, die von Tabletten zwischen 2,5 und 30,0 mm. Der Wirkstoffgehalt kann je nach eingesetztem Wirkstoff und gewünschter Freisetzungsgeschwindigkeit in weiten Grenzen variieren. So kann der Wirkstoffgehalt im Bereich von 0,1 bis 98 Gew.%, vorzugsweise von 50 bis 80 Gew.% bezogen auf das Gesamtgewicht des Kerns liegen.

Der wirkstoffhaltige Kern kann ein Wirkstoffpellet oder Wirkstoffgranulat sein, welches den/die Wirkstoff(e) und pharmazeutisch übliche Hilfsstoffe enthält. Hierzu werden Wirkstoff(e) und Hilfstoffe gemeinsam granuliert.

Der wirkstoffhaltige Kern in Form von Pellets oder Mikropellets kann einen "inerten Kern" enthalten, der mit einer wirkstoffhaltigen Schicht überzogen ist. Der "inerte Kern" kann aus einem wasserunlöslichen Material bestehen, beispielsweise Glas, Cellulose (z. B. mikrokristalline Cellulose), Oxide und/oder organische Polymere. Als organische Polymere eignen sich Polypropylen oder Polyethylen. Er kann auch aus wasserlöslichem Material aufgebaut sein, wie anorganische Salze, Zucker oder non-pareils. Diese "inerten Kerne" können einen Durchmesser von 10 bis 2000 µm, bevorzugt von 50 bis 500 µm haben.

Das inerte Kernmaterial kann mit dem (den) Wirkstoff (en) in Form von Kristallen, Agglomeraten, etc. beschichtet sein. Die Wirkstoff-Beschichtung der inerten Kerne kann beispielsweise mit Hilfe von Granulierung oder Spray-Coating erfolgen. Die Größe der wirkstoffhaltigen Kerne beträgt 200 bis 2000 µm, bevorzugt 200 bis 800 µm.

Vor Beschichtung des inerten Kernmaterials kann der (die) Wirkstoff(e) mit Hilfsstoffen gemischt werden, beispielsweise Bindemittel, Tenside, Sprengmittel und/oder andere pharmazeutisch akzeptable Hilfsstoffe. Als Bindemittel können Cellulosen, wie Hydroxypropylmethylcellulose, Hydroxypropylcellulose oder Natriumcarboxymethylcellulose, Polyvinylpyrrolidon, Zucker und/oder Stärke eingesetzt werden. Als Tenside eignen sich nicht-ionische oder ionische oberflächenaktive Substanzen, wie z.B. Natriumlaurylsulfat.

Der wirkstoffhaltige Kern kann eine Tablette oder Minitablette (Durchmesser kleiner 4 mm) sein. Diese Kerne können neben dem Wirkstoff weitere pharmazeutische Hilfsstoffe wie Trägermaterialien, Füllstoffe, Bindemittel, Feuchthaltemittel, Zerfallsföderer, Sprengmittel, Schmiermittel, Fließregulierungsmittel, Formentrennmittel, Konservierungsmittel, Aromen und/oder Farbpigmente enthalten. Übliche Herstellungsverfahren sind direktes Verpressen oder Verpressen von Trocken-, Feucht- oder Sintergranulaten.

### Überzug:

Die wirkstoffhaltigen Kerne können mit einer einzigen Schicht überzogen werden, die ein oder mehrere filmbildende Polymere und mindestens zwei Trennmittel enthält.

Als filmbildende Polymere auf Wasserbasis eignen sich Polyacrylate.

Mit dem Begriff "Polyacrylat" werden beispielsweise Copolymerisate mit zwei oder mehr Monomeren wie Acrylsäure, Methacrylsäure, Acrylatester oder Methacrylatester, wie beispielsweise Aminoalkylester oder Alkylester, insbesondere Methyl-, Ethyl-, Propyl-, Butylester als auch hydroxylierte Acryl- ober Methacrylsäureester bezeichnet. Als filmbildende Polyacrylate eignen sich beispielsweise Polyethylacrylatmethylmethacrylate, Polyethylacrylatmethacrylsäure, Polymethacrylsäuremethylmethacrylate und/oder Copolymerisate aus Acryl- und Methacrylsäureestern mit quartären Ammoniumgruppen.

Polyacrylate sind unter dem Handelsnamen "Eudragit" der Fa. Röhm erhältlich. Erfindungsgemäß können Eudragit NE, Eudragit RL, Eudragit RS, Eudragit L, Eudragit S, Eudragit FS oder deren Mischungen eingesetzt werden. Bevorzugt wird Eudragit NE30D verwendet.

Bei Eudragit NE30D handelt es sich um Polyethylacrylatmethylmethacrylat in Form einer 30%igen wässrigen Dispersion, mit einem Verhältnis der Copolymeren von 2:1. Eudragit NE zeigt eine Filmbildungstemperatur von 5°C (Minimum). Das Copolymerisat hat einen neutralen Charakter und ist wasserunlöslich im gesamten pH-Bereich des Verdauungstrakts. Eudragit NE zeigt eine pH-unabhänige Permeabilität. Der Überzug eines wirkstoffhaltigen Kerns mit Eudragit NE führt demnach zu einer diffusionskontrollierten Retardierung der Wirkstofffreisetzung, da das Eudragit in Wasser quillt. Für die Verarbeitung von Eudragit NE30D ist ein Zusatz von Weichmachern nicht erforderlich.

Eine ähnliche Dispersion wie Eudragit NE30D ist Kollicoat EMM30D der Fa. BASF.

Eine wässrige Dispersion mit 40% Polymeranteil liegt bei Eudragit NE40D vor.

Bei Eudragit RL und RS handelt es sich um Copolymerisate aus Acryl- und Methacrylsäureestern mit einem geringen Gehalt an quartären Ammoniumgruppen und zwar um Poly(ethylacrylatmethylmethacrylattrimethylammoniumethylmethylmethacrylatchlorid). Das Verhältnis der Copolymeren beträgt 1:2:0.2 bei Eudragit RL bzw. 1:2:0.1 bei Eudragit RS. Die Copolymerisate sind wasserunlöslich im gesamten pH-Bereich des Verdauungstrakts und zeigen eine pH-unabhänige Permeabilität.

Eudragit RS, ein schwach kationisches hydrophiles Polymethacrylat benötigt einen Weichmacherzusatz von 10 bis 20%, um die Filmbildungstemperatur unter 20°C zu senken. Als Weichmacher eignen sich Acetyltriethylcitrat, Diethylsebacat, Dibutylsebacat, Diethylphthalat, Dibutylphthalat, Triacetin, 1,2-Propylenglycol, Polyethylenglycol 6000 oder insbesondere Triethylcitrat. Eudragit RS30D liegt als 30%ige wässrige Dispersion vor.

Eudragit RL 30D benötigt einen Weichmacherzusatz von ca. 20 %. Als Weichmacher eignen sich Acetyltriethylcitrat, Diethylsebacat, Triacetin, 1,2-Propylenglycol oder insbesondere Triethylcitrat.

Eudragit FS30D ist eine 30%ige Dispersion enthaltend ein Copolymer aus 65 Gew.-% Methylacrylat, 25 Gew.-% Methylmethacrylat und 10 Gew.-% Methacrylsäure.

Bei Eudragit L30D55 handelt es sich um eine 30%ige wässrige Dispersion eines Copolymerisats mit anionischem Charakter auf Basis von Methacrylsäure und Ethylacrylat. Das Verhältnis der freien Carboxylgruppen zu den Estergruppen beträgt etwa 1:1. Eudragit L30D55 ist für magensaftresistente Überzüge geeignet und ab pH=5,5 darmsaftlöslich. Eudragit L30D55 benötigt einen Weichmacherzusatz von ca. 10 bis 15%. Als Weichmacher eignen sich Triethylcitrat und Polyethylenglycol.

Eudragit S ist ein Copolymerisat mit anionischem Charakter auf Basis von Methacrylsäure und Methylmethacrylat. Das Verhältnis der freien Carboxylgruppen zu den Estergruppen beträgt etwa 1:2. Eudragit S ist für magensaftresistente Überzüge geeignet und ab pH=7 darmsaftlöslich. Das Copolymerisat zeigt eine pHabhängige Retardierung. Eudragit S benötigt einen Weichmacherzusatz, beispielsweise Triethylcitrat und Polyethylenglycol.

Der Gehalt an Polymer(en) kann 40 bis 90 Gew.% bezogen auf den gesamten Überzug betragen. Bevorzugt wird ein Gehalt von 60 bis 70 Gew.%. Das(die) Polymer(en) kann (können) zusammen mit einer Mischung aus zwei oder mehreren Trennmitteln eingesetzt werden, wobei mindestens ein Trennmittel ein Fettsäuresalz und mindestens ein weiteres Trennmittel ein Schichtsilikat sein kann.

Als Fettsäuresalz eignen sich beispielsweise Alkali-, Erdalkali- oder Aluminiumsalze von Fettsäuren, wie Natrium-, Kalium-, Magnesium-, Calcium-, Aluminiumstearat, oder Natrium-, Kalium-, Magnesium- oder calciumbehenat oder Magnesiumsalze der Caprylsäure, Caprinsäure, Laurinsäure oder Palmitinsäure.

Als Schichtsilicate eignen sich Talk, Kaolinit, Pyrophyllit, Attapulgit, Sepiolit, Muskovit, Montmorillonit, Bentonit und/oder Vermiculit.

Der Gehalt an einem oder an mehreren Schichtsilikaten kann 20 bis 60 Gew.% bezogen auf das Trockengewicht des/der Polymer(en) betragen. Bevorzugt wird ein Gehalt von 30 bis 50 Gew.%.

Der Gehalt an einem oder an mehreren Fettsäuresalzen kann 5 bis 40 Gew.% bezogen auf das Trockengewicht des/der Polymer(en) betragen. Bevorzugt wird ein Gehalt von 10 bis 30 Gew.%.

Als weitere Hilfsstoffe können beispielsweise hydrophile Komponenten (z.B. Aerosil oder Polyethylenglycole) eingesetzt werden.

Ein Zusatz von Stabilisatoren, wie Tenside (z. B. nicht-ionische Tenside wie Polysorbat, Sorbitan-monoisostearat, Sorbitan-monolaurat, Sorbitan-monopalmitat, Sorbitan-monostearat, Sorbitan-monooeleat, Sorbitan-sesquioleat, Sorbitan-trioleat, Glycerylmonostearat, Glycerylmonooleat, Polyvinylalkohol oder anionische Tenside wie Natriumdocusat, Natriumlaurylsulfat oder kationische Tenside wie Benzalkoniumchlorid, Benzethoniumchlorid oder Cetrimid) oder Antischaummittel (z. B. Schaumverhütungsmittel auf Siliconbasis wie Polydimethylsiloxane, Simethicon®, Dimethicon®, Silicon-Emulsion bzw. Glycerol, Sorbitol oder PEG-Derivate ist nicht erforderlich.

Die Polymeren können als wässrige Dispersion verarbeitet werden. Es müssen keine organischen, mit Wasser mischbaren Lösungsmitteln wie niedere Alkohole, beispielsweise Ethanol, Propanol, Isopropanol zugesetzt werden.

Die Schichtdicke des Polymer-Überzugs beträgt bevorzugt 25 bis 75 µm.

Werden Pellets mit der erfindungsgemäßen Polymer-Dispersion überzogen, dann kann der Durchmesser der überzogenen Pellets 10 bis 2000 µm betragen. Unter Mikropellets versteht man Pellets mit einem Durchmesser kleiner als 1000 µm. Die erfindungsgemäßen, überzogenen Mikropellets haben bevorzugt einen Durchmesser von 300 bis 800 µm.

### Verfahren:

Bei dem erfindungsgemäßen Verfahren zur Herstellung von überzogenen Formulierungen werden zunächst mindestens ein Fettsäuresalz und mindestens ein Schichtsilikat gemischt. Das Mischen kann bei Raumtemperatur mit Hilfe von Mischapparaturen wie Freifall- oder Pflugscharmischern, beispielsweise in einem Turbula- oder Lödige-Mischer erfolgen. Die Trennmittel-Mischung kann unter Rühren zu einer wässrigen Suspension des (der) Polymer(en) gegeben werden. Es können weitere Hilfsstoffe zugesetzt werden. Die so erhaltene Polymer-Suspension kann auf die wirkstoffhaltigen Kerne aufgesprüht werden. Das Aufsprühen der wässrigen Polymer-Suspension kann mittels "Coating pans", Wirbelschicht-, Accela-cota-, Tauchrohr-, Tauchschwertverfahren oder Kesseldragierung erfolgen.

Das Besprühen von Tabletten, Minitabletten oder Kapseln kann in einem Wirbelschichtgerät, einem Drageekessel oder einer Lackieranlage mit perforierter Trommel, Zuluft-/Abluftversorgung und Sprühvorrichtung erfolgen.

Für das Besprühen von Wirkstoff-Kristallen, Granulaten, Pellets oder Mikropellets können Wirbelschichtgeräte vom Typ "top-spray", "Wurster-bottom-spray" oder "tangential-spray" eingesetzt werden (vgl. auch "Air suspension coating for multiparticulates", D. Jones, Drug Development and Industrial Pharmacy, 20 (20), 1994, S. 3175-3206) .

Besonders bevorzugt wird für Mikropellets ein Wirbelschichtgerät der Fa. Glatt mit Wurster-Insert verwendet. Ein Düsendurchmesser von 0.8 bis 2.0 mm, eine Sprührate von 20 bis 600 ml/min., ein Sprühdruck von 1.0 bis 2.7 bar sowie eine Produkttemperatur von 22 °C bis 26°C während des Sprühprozesses sind vorteilhaft. Das Trocknen der Mikropellets kann im Wirbelschichtgerät vorzugsweise bei einer Produkttemperatur von 25 bis 30°C erfolgen. Nach dem Sieben erhält man fließfähige Mikropellets mit einheitlicher Korngrößenverteilung.

### Weiterverarbeitung zu Kapseln:

Die überzogenen, wirkstoffhaltigen Pellets, Mikropellets, Granulate oder Minitabletten können in Kapseln gefüllt werden. Als Kapseln eignen sich Hart- oder Weichgelatinekapseln.

### Weiterverarbeitung zu Tabletten:

Um eine multiple-unit-dosage form in Form einer Tablette zu erhalten, können überzogene, wirkstoffhaltige Mikropellets mit Hilfsstoffen gemischt und zu Tabletten verpresst werden.

Als Hilfsstoffe bei der Tablettenherstellung eignen sich:
- Füllstoffe wie Cellulose und/oder Cellulosederivate (z. B. mikrokristalline Cellulose), Zucker (z. B. Lactose, Glucose, Saccharose), Zuckeralkohole (z. B. Mannit, Sorbit), Stärke (z. B. Kartoffel-, Weizen-, Mais- und/oder Reisstärke),
- Schmiermittel wie Magnesiumstearat, Calciumstearat, Stearinsäure, hydrierte Pflanzenöle und/oder Talkum,
- Fließregulierungsmittel wie hochdisperses Siliciumdioxid,
- Sprengmittel wie Stärke und -derivate (Natriumcarboxymethylstärke), quervernetztes Polyvinylpyrrolidon, unmodifizierte oder modifizierte Cellulose (z. B. Natriumcarboxymethylcellulose, quervernetzte Carboxymethylcellulose) und/oder Alginate.

Die Tabletten können mit einem filmbildenden Material überzogen werden, um eine glatte Oberfläche zu erhalten oder um die Stabilität der Tablette während der Verpackung und dem Transport zu erhöhen. Dieser Tablettenüberzug kann beispielsweise Additive wie "anti-tacking" Materialien oder Farbstoffe enthalten.

Der Gehalt der Mikropellets kann maximal 70% des gesamten Tablettengewichts betragen. Bevorzugt wird ein Gehalt von 25 bis 55%.

### Freisetzungsprofil:

In-vitro Freisetzungsstudien für die beschriebenen Formulierungen wurden mit einer USP Standard-Apparatur mit simuliertem Magensaft (pH = 1,2) oder im Puffermedium (pH = 6,8) durchgeführt.

Die Erfindung wird durch nachstehende Beispiele näher erläutert, ohne aber den Erfindungsumfang damit einzuschränken.

### Beispiel 1

Die folgenden Stoffe werden zur Herstellung von Morphinsulfat-Tabletten verwendet.

| **Bestandteile** | **Prozent** | **Gewicht (mg/Tablette)** |
|---|---|---|
| Morphinsulfat | 2,3 | 10,0 |
| Cellulosepellets | 21,0 | 90,0 |
| Eudragit NE30D | 10,3* | 44,5* |
| Talkum | 6,0 | 25,7 |
| Calciumstearat | 4,1 | 17,6 |
| Lactose | 49,3 | 212,0 |
| Stearinsäure | 1,0 | 4,2 |
| Natriumcarboxymethyl-cellulose | 3,0 | 12,9 |
| Hochdisperses Siliciumdioxid | 0,5 | 2,1 |
| Hydroxypropylmethyl-cellulose | 2,1 | 9,0 |
| PEG 8000 | 0,2 | 1,0 |
| Eisenoxid gelb | 0,2 | 1,0 |
| Gesamt | 100,0 | 430,0 |

| | | |
|---|---|---|
| * angegeben ist die Menge an Lacktrockensubstanz | | |

### Herstellung:

Die Cellulosepellets werden zunächst mit einer wässrigen Lösung von Morphinsulfat besprüht. Die wirkstoffhaltigen Kerne werden anschließend mit einer Schicht aus Eudragit NE30D/Talkum/Calciumstearat überzogen. Die überzogenen Pellets werden anschließend mit Lactose (Füllstoff), Stearinsäure (Schmiermittel), Natriumcarboxymethylcellulose (Sprengmittel) sowie hochdispersem Siliciumdioxid (Fließregulierungsmittel) gemischt und zu Tabletten verpresst. Anschließend werden die Tabletten mit einem wasserlöslichen HPMC-Lack, der PEG 8000 als Weichmacher und Eisenoxid als Farbpigment enthält, befilmt.

### Beispiel 2

Die folgenden Stoffe werden zur Herstellung von Metoprololsuccinat Tabletten verwendet.

| **Bestandteile** | **Prozent** | **Gewicht (mg/Tablette)** |
|---|---|---|
| Metoprololsuccinat | 15,8 | 95,0 |
| Zuckerpellets | 27,0 | 162,2 |
| Eudragit NE30D | 13,4* | 80,2* |
| Talkum | 3,0 | 18,3 |
| Magnesiumstearat | 0,7 | 4,3 |
| Mikrokristalline Cellulose | 36,5 | 219,0 |
| Crospovidon | 3,4 | 20,2 |
| Hochdisperses Siliciumdioxid | 0,2 | 0,8 |
| Gesamt | 100,0 | 600,0 |

| | | |
|---|---|---|
| * angegeben ist die Menge an Lacktrockensubstanz | | |

### Herstellung:

Die Zuckerpellets werden zunächst mit einer wässrigen Lösung von Metoprololsuccinat besprüht. Die wirkstoffhaltigen Kerne werden anschliessend mit einer Schicht aus Eudragit NE30D/Talkum/Magnesiumstearat überzogen. Die überzogenen Pellets werden zusammen mit weiteren Hilfsstoffen zu Tabletten verpresst.

### Vergleich der Freisetzungsprofile

Vergleich des Freisetzungsprofils einer Metropololsuccinat-Tablette, die Metoprololsuccinat-haltige Kerne überzogen mit Eudragit NE30D, Magnesiumstearat und Talk enthält, mit einer entsprechenden Formulierung, bei der nur Talk als Trennmittel verwendet wurde.

Apparatur zur Bestimmung der Wirkstofffreisetzung:

| | |
|---|---|
| Medium: | KH₂PO₄-Puffer pH 6,8, 900ml, Paddle |
| Temperatur: | 37°C |
| Rührgeschwindigkeit: | 100 UpM |

| | **Überzug mit Eudragit NE30D/Talk/Magnesiumstearat** | **Überzug mit Eudragit NE30D/Talk** |
|---|---|---|
| Zeit (min) | Freigesetzter Wirkstoff (%) | Freigesetzter Wirkstoff (%) |
| 120 | 18 | 5 |
| 360 | 44 | 25 |
| 600 | 65 | 60 |
| 840 | 80 | 78 |
| 1200 | 95 | 85 |

Wie man der Tabelle entnehmen kann, erhält man bei Verwendung von Talk und Magnesiumstearat als Trennmittel ein Freisetzungsprofil 0. Ordnung. Bei Verwendung von Talk alleine ergibt sich ein Freisetzungprofil 1. Ordnung.

### Beispiel 3

Die folgenden Stoffe werden zur Herstellung von Bisoprololfumarat Tabletten verwendet.

| **Bestandteile** | **Prozent** | **Gewicht (mg/Tablette)** |
|---|---|---|
| Bisoprololfumarat | 2,4 | 10,0 |
| Cellulosepellets | 46,3 | 190,0 |
| Eudragit NE30D | 10,2* | 42,0* |
| Bentonit | 4,9 | 20,0 |
| Aluminiumstearat | 2,0 | 8,0 |
| Mikrokristalline Cellulose | 21,1 | 86,5 |
| Lactose | 9,1 | 37,1 |
| Natriumcarboxymethylcel lulose | 3,0 | 12,3 |
| Hochdisperses Siliciumdioxid | 1,0 | 4,1 |
| Gesamt | 100,0 | 410,0 |

| | | |
|---|---|---|
| * angegeben ist die Menge an Lacktrockensubstanz | | |

### Herstellung:

Die Cellulosepellets werden zunächst mit einer wässrigen Lösung von Bisoprololfumarat besprüht. Die wirkstoffhaltigen Kerne werden anschliessend mit einer Schicht aus Eudragit NE30D/Bentonit/Aluminiumstearat überzogen. Die überzogenen Pellets werden zusammen mit weiteren Hilfsstoffen zu Tabletten verpresst.

### Beispiel 4

Die folgenden Stoffe werden zur Herstellung von Tramadol-Hydrochlorid-Kapseln verwendet.

| **Bestandteile** | **Prozent** | **Gewicht (mg/Kapsel)** |
|---|---|---|
| Tramadol-Hydrochlorid | 28,2 | 100,0 |
| Zuckerpellets | 28,2 | 100,0 |
| Eudragit NE30D | 18,0* | 64,0* |
| Talkum | 3,6 | 12,8 |
| Calciumbehenat | 0,9 | 3,2 |
| Hartgelatinekapsel | 21,3 | 76,0 |
| Gesamt | 100,0 | 356,0 |

| | | |
|---|---|---|
| * angegeben ist die Menge an Lacktrockensubstanz | | |

### Herstellung:

Die Zuckerpellets werden zunächst mit einer wässrigen Lösung von Tramadolhydrochlorid besprüht. Die wirkstoffhaltigen Kerne werden anschliessend mit einer Schicht aus Eudragit NE30D/Talkum/Calciumbehenat überzogen. Die überzogenen Pellets werden in Hartgelatinekapseln gefüllt.

### Beispiel 5

Die folgenden Stoffe werden zur Herstellung von Oxycodon-Hydrochlorid-Kapseln verwendet.

| **Bestandteile** | **Prozent** | **Gewicht (mg/Kapsel)** |
|---|---|---|
| Oxycodon-Hydrochlorid | 3,1 | 10,0 |
| Mikrokristalline Cellulose | 47,8 | 155,7 |
| Magnesiumstearat | 0,5 | 1,7 |
| Aerosil | 0,8 | 2,6 |
| Eudragit NE30D | 15,3* | 50,0* |
| Kaolin | 6,1 | 20,0 |
| Magnesiumstearat | 3,1 | 10,0 |
| Hartgelatinekapsel | 23,3 | 76,0 |
| Gesamt | 100,0 | 326,0 |

| | | |
|---|---|---|
| * angegeben ist die Menge an Lacktrockensubstanz | | |

### Herstellung:

Oxycodon-Hydrochlorid wird zusammen mit Mikrokristalliner Cellulose, Aerosil und Magnesiumstearat zu Minitabletten verpresst. Die Minitabletten werden anschliessend mit einer Schicht aus Eudragit NE30D/Kaolin/Magnesiumstearat überzogen. Die überzogenen Minitabletten werden in Hartgelatinekapseln gefüllt.

## Patentansprüche

1. Verfahren zur Herstellung einer wässerigen Dispersion zur Herstellung eines Überzuges, umfassend die Schritte, dass zunächst mindestens ein Fettsäuresalz und mindestens ein Schichtsilikat gemischt werden, um eine Trennmittel-Mischung herzustellen, und dann die Trennmittel-Mischung zu einer wässerigen Suspension von (einem) filmbildenden Polymer(en) zugegeben wird.

2. Verfahren nach Anspruch 1, wobei es sich bei dem filmbildenden Polymeren um ein Gemisch von filmbildenden Polymeren handelt.

3. Verfahren nach einem der vorhergehenden Ansprüche, mit einem Polyacrylat als filmbildenden Polymeren.

4. Verfahren nach Anspruch 3, wobei es sich bei dem Polyacrylat um ein Polymerisat auf Basis von Acrylsäure, Methacrylsäure, Acrylsäureester und/oder Methacrylsäureester handelt.

5. Verfahren nach einem der vorhergehenden Ansprüche, wobei das Fettsäuresalz ein Alkalimetallsalz und/oder ein Erdalkalimetallsalz und/oder ein Aluminiumsalz einer Fettsäure ist.

6. Verfahren nach Anspruch 5, wobei das Alkalimetallsalz und/oder Erdalkalimetallsalz ein Natrium-, Kalium-, Magnesium-und/oder Calciumbehenat-Salz ist.

7. Verfahren nach Anspruch 5, wobei das Alkalimetallsalz und/oder Erdalkalimetallsalz ein Natrium-, Kalium-, Magnesium-, Calcium- und/oder Aluminiumstearat-Salz ist.

8. Verfahren nach Anspruch 5, wobei das Magnesiumsalz ein Salz der Caprylsäure, Caprinsäure, Laurinsäure und/oder Palmitinsäure ist.

9. Verfahren nach einem der vorhergehenden Ansprüche, wobei der Gehalt an Fettsäuresalz 5 bis 40 Gew.-% beträgt, vorzugsweise 10 bis 30 Gew.-%, jeweils bezogen auf das Trockengewicht des filmbildenden Polymeren.

10. Verfahren nach einem der vorhergehenden Ansprüche, wobei die Mischung als Schichtsilikat Talk, Kaolinit, Pyrophyllit, Attapulgit, Sepolit, Muskovit, Montmorillonit, Bentonit und/oder Vermiculit umfasst.

11. Verfahren nach einem der vorhergehenden Ansprüche, wobei der Gehalt an Silikat 20 bis 60 Gew.-% beträgt, vorzugsweise 30 bis 50 Gew.-%, jeweils bezogen auf das Trockengewicht des filmbildenden Polymeren.

12. Verfahren zur Herstellung einer pharmazeutischen wirkstoffhaltigen Formulierung, umfassend das Aufbringen der wässerigen Dispersion zur Herstellung eines Überzuges, welche durch das Verfahren nach einem der vorhergehenden Ansprüche hergestellt wird, durch Aufsprühen auf einen wirkstoffhaltigen Kern.

13. Verfahren nach Anspruch 12, wobei der Schritt des Aufbringens durch Aufsprayen mittels "coating pans", Wirbelschicht-, Accela-cota-, Tauchrohr-, Tauchschwertverfahren oder Kesseldragierung erfolgt.

14. Verfahren nach einem der vorhergehenden Ansprüche, wobei der wirkstoffhaltige Kern ausgewählt wird aus Kapseln, Tabletten, Pellets, Granulaten, Minitabletten und Mikropellets.

15. Verfahren nach einem der vorhergehenden Ansprüche, wobei der wirkstoffhaltige Kern ein Wirkstoff-Kristall ist.

16. Verfahren nach Anspruch 14, wobei ein Pellet oder Mikropellet als wirkstoffhaltiger Kern einen inerten Kern (Inert-Kern) umfasst, wobei insbesondere ein inerter Kern mit einem wirkstoffhaltigen Überzug zu einem wirkstoffhaltigen Kern ausgebildet ist.

17. Verfahren nach Anspruch 14 und/oder 16, wobei die Mikropellets als Miltiple-Unit-Dosage-Form vorgesehen sind, insbesondere in Form von Tabletten oder in Kapseln.

18. Verfahren nach Anspruch 14 und/oder 16, wobei die Pellets, Granulate oder Minitabletten als Multiple-Unit-Dosage-Form vorgesehen sind, insbesondere in Kapseln.

19. Verfahren nach Anspruch 17 und/oder 18, wobei die Multiple-Unit-Dosage-Form ihrerseits mit einem Überzug durch ein Verfahren gemäß mindestens einem der Ansprüche 1 bis 13 versehen wird.

20. Verfahren nach mindestens einem der Ansprüche 17 bis 18, wobei es sich bei der Multiple-Dosage-Form um eine Kapsel handelt, insbesondere eine Weichgelantine-Kapsel.

21. Verfahren nach einem der vorhergehenden Ansprüche, wobei der Wirkstoff im Gemisch mit pharmazeutisch akzeptablen Hilfsstoffen vorgesehen ist, insbesondere mit üblichen Hilfsstoffen.

22. Verfahren nach einem der vorhergehenden Ansprüche, wobei der Wirkstoff im Gemisch mit Tensiden, insbesondere nicht-ionischen oder ionischen oberflächenaktiven Substanzen, vorgesehen oder frei von Tensiden ist.

23. Verfahren nach einem der vorhergehenden Ansprüche, wobei der Wirkstoff ausgewählt wird aus Metoprolol, Bisoprolol, Tramadol, Morphin, Oxycodon und Hydrocodon, einschließlich Stereoisomere und pharmazeutisch unbedenkliche Salze, Hydrate und Solvate davon.

24. Verfahren nach einem der vorhergehenden Ansprüche, wobei der Wirkstoff Metoprolol oder eines seiner Salze ist, insbesondere Metoprolol-succinat.

## Claims

1. A process for the preparation of an aqueous dispersion for the preparation of a coating, comprising the steps of:
initially mixing together at least one fatty acid salt and at least one layer silicate to form a separation agent mixture, and then adding the separating agent mixture to an aqueous suspension of (a) film-forming polymer(s).

2. The process according to claim 1, wherein the film-forming polymer is a mixture of film-forming polymers.

3. The process according to any one of the preceding claims, having a polyacrylate as film-forming polymer.

4. The process according to claim 3, wherein the polyacrylate is a polymer based on acrylic acid, methacrylic acid, acrylic acid ester and/or methacrylic acid ester.

5. The process according to any one of the preceding claims, wherein the fatty acid salt is an alkali metal salt and/or an alkaline earth metal salt and/or an aluminium salt of a fatty acid.

6. The process according to claim 5, wherein the alkali metal salt and/or alkaline earth metal salt is a sodium, potassium, magnesium and/or calcium behenate salt.

7. The process according to claim 5, wherein the alkali metal salt and/or alkaline earth metal salt is a sodium, potassium, magnesium, calcium and/or aluminium stearate salt.

8. The process according to claim 5, wherein the magnesium salt is a salt of caprylic acid, capric acid, lauric acid and/or palmitic acid.

9. The process according to any one of the preceding claims, wherein the content of fatty acid salt is from 5 to 40 % by weight, preferably from 10 to 30 % by weight, in each case based on the dry weight of the film-forming polymer.

10. The process according to any one of the preceding claims, wherein the mixture comprises talcum, kaolinite, pyrophyllite, attapulgite, sepolite, muscovite, montmorillonite, bentonite and/or vermiculite as layer silicate.

11. The process according to any one of the preceding claims, wherein the content of silicate is from 20 to 60 % by weight, preferably from 30 to 50 % by weight, in each case based on the dry weight of the film-forming polymer.

12. A process for the preparation of a pharmaceutical active-ingredient-containing formulation comprising applying the aqueous coating dispersion manufactured by the process of any one of the preceding claims to an active-ingredient-containing core by spraying.

13. The process according to claim 12, wherein the spray application step can be carried out using coating pans, fluidised bed, Accela-coater, dip tube or dip blade processes or pen coating.

14. The process according to any one of the preceding claims, wherein the active-ingredient-containing core is selected from capsules, tablets, pellets, granules, minitablets or micropellets.

15. The process according to any one of the preceding claims, wherein the active-ingredient-containing core is an active ingredient crystal.

16. The process according to claim 14, wherein a pellet or micropellet as active-ingredient-containing core comprises an inert core, an active-ingredient-containing core especially being constituted by an inert core with an active-ingredient-containing coating.

17. The process according to claim 14 and/or 16, wherein the micropellets are provided as multiple-unit-dosage form, especially in the form of tablets or in capsules.

18. The process according to claim 14 and/or 16, wherein the pellets, granules or minitablets are provided as multiple-unit-dosage form, especially in capsules.

19. The process according to claim 17 and/or 18, wherein the multiple-unit-dosage form is in turn provided with a coating by a process according to at least one of claims 1 to 13.

20. The process according to at least one of the claims 17 to 18, wherein the multiple-dosage form is a capsule, especially a soft gelatine capsule.

21. The process according to any one of the preceding claims, wherein the active ingredient is provided in admixture with pharmaceutically acceptable auxiliaries, especially with customary auxiliaries.

22. The process according to any one of the preceding claims, wherein the active ingredient is provided in admixture with surfactants, especially non-ionic or ionic surface-active substances, or is free of surfactants.

23. The process according to any one of the preceding claims, wherein the active ingredient is selected from metoprolol, bisoprolol, tramadol, morphine, oxycodon and hydrocodon, including stereoisomers and pharmaceutically acceptable salts, hydrates and solvates thereof.

24. The process according to anyone of the proceeding claims, wherein the active ingredient is metoprolol or a salt thereof, especially metoprolol succinate.

## Revendications

1. Procédé de fabrication d'une dispersion aqueuse pour la fabrication d'un enrobage, comprenant les étapes, au moins un sel d'acide gras et au moins un silicate en feuillets sont d'abord mélangés afin de fabriquer un agent de séparation, et ensuite l'agent de séparation est ajouté à une suspension aqueuse d'un (de) polymère(s) filmogène(s).

2. Procédé selon la revendication 1, où il s'agit d'un mélange de polymères filmogènes dans le cas du polymère filmogène.

3. Procédé selon l'une des revendications précédentes comprenant un polyacrylate en tant que polymère filmogène.

4. Procédé selon la revendication 1, où il s'agit d'un produit de polymérisation à base d'acide acrylique, d'acide méthacrylique, d'ester d'acide acrylique et/ou d'ester d'acide méthacrylique dans le cas du polyacrylate.

5. Procédé selon l'une des revendications précédentes où le sel d'acide gras est un sel de métal alcalin et/ou un sel de métal alcalino-terreux et/ou un sel d'aluminium.

6. Procédé selon la revendication 5 où le sel de métal alcalin et/ou le sel de métal alcalino-terreux est un sel béhénate de sodium, de potassium, de magnésium et/ou de calcium.

7. Procédé selon la revendication 5 où le sel de métal alcalin et/ou le sel de métal alcalino-terreux est un sel stéarate de sodium, de potassium, de magnésium, de calcium et/ou d'aluminium.

8. Procédé selon la revendication 5 où le sel de magnésium est un sel de l'acide caprylique, de l'acide caprinique, de l'acide laurique et/ou de l'acide palmitique.

9. Procédé selon l'une des revendications précédentes, la teneur en sel d'acide gras étant de 5 à 40 % en poids, de préférence de 10 à 30 % en poids, chaque fois par rapport au poids à sec du polymère filmogène.

10. Procédé selon l'une des revendications précédentes, le mélange de phyllosilicates comprenant du talc, de la kaolinite, de la pyrophyllite, de l'attapulgite, de la sépiolite, de la muscovite, de la montmorillonite, de la bentonite et/ou de la vermiculite.

11. Procédé selon l'une des revendications précédentes, la teneur en silicate étant de 20 à 60 % en poids, de préférence de 30 à 50 % en poids, chaque fois par rapport au poids à sec du polymère filmogène.

12. Procédé de fabrication d'une formulation pharmaceutique contenant une substance active comprenant la mise en oeuvre de la dispersion aqueuse pour la fabrication d'un enrobage, dispersion qui est fabriquée par le procédé selon l'une des revendications précédentes, par pulvérisation sur un coeur contenant la substance active.

13. Procédé selon la revendication 12 où l'étape de l'apport par pulvérisation est réalisée au moyen de procédés de dragéification par « coating pans », enrobage en lit fluidisé, pelliculage Accela-cota, enrobage par tube plongeur, enrobage par immersion ou dragéification dans une turbine.

14. Procédé selon l'une des revendications précédentes où le coeur contenant la substance active est choisi parmi des capsules, des comprimés, des pastilles, des granulés, des mini comprimés ou des micro pastilles.

15. Procédé selon l'une des revendications précédentes où le coeur contenant la substance active est un cristal de substance active.

16. Procédé selon la revendication 14 dans lequel une pastille ou une micro pastille servant de coeur contenant la substance active comprend un coeur inerte (coeur inerte), en particulier, un coeur inerte avec un pelliculage contenant la substance active étant transformé en un coeur contenant la substance active.

17. Procédé selon les revendications 14 et/ou 16, la forme de l'unité de dosage multiple étant prévue sous la forme de micro pastilles, en particulier sous forme de comprimés ou de capsules.

18. Procédé selon les revendications 14 et/ou 16, la forme de l'unité de dosage multiple étant prévue sous la forme de pastilles, de granulés ou de mini comprimés, en particulier sous la forme de capsules.

19. Procédé selon les revendications 17 et/ou 18 où, pour sa part, la forme de l'unité de dosage multiple est munie d'un enrobage par un procédé selon au moins l'une des revendications de 1 à 13.

20. Procédé selon au moins l'une des revendications de 17 à 18 où il s'agit d'une capsule, en particulier d'une capsule en gélatine molle, dans le cas de la forme de dosage multiple.

21. Procédé selon l'une des revendications précédentes, où la substance active est prévue être en mélange avec des produits auxiliaires pharmaceutiquement acceptables, en particulier, avec des produits auxiliaires usuels.

22. Procédé selon l'une des revendications précédentes où il est prévu que la substance active soit en mélange avec des tensio-actifs, en particulier des substances tensioactives non ioniques ou ioniques, ou soit exempte de tensioactifs.

23. Procédé selon l'une des revendications précédentes où la substance active est choisie parmi du métoprolol, du bisoprolol, du tramadol, de la morphine, de l'oxycodone et de l'hydrocodone, y compris leurs stéréo-isomères et leurs sels ne présentant pas d'inconvénient d'un point de vue pharmaceutique, leurs hydrates et leurs solvates.

24. Procédé selon l'une des revendications précédentes où la substance active est le métoprolol ou l'un de ses sels, en particulier le succinate de métoprolol.
